Europäisches Patentamt

European Patent Office

Office européen des brevets

(19)

(11) Veröffentlichungsnummer: **0 152 068 B1**

(12) **EUROPÄISCHE PATENTSCHRIFT**

(45) Veröffentlichungstag der Patentschrift: 20.03.91

(51) Int. Cl.⁵: **G01N 31/22**

(21) Anmeldenummer: 85101299.7

(22) Anmeldetag: 07.02.85

(54) **Verfahren zum Nachweis von allergenhaltigem Hausstaub.**

(30) Priorität: 10.02.84 DE 3404821

(43) Veröffentlichungstag der Anmeldung:
21.08.85 Patentblatt 85/34

(45) Bekanntmachung des Hinweises auf die
Patenterteilung:
20.03.91 Patentblatt 91/12

(84) Benannte Vertragsstaaten:
**AT BE CH FR GB IT LI LU NL SE**

(56) Entgegenhaltungen:
FR-A- 2 502 786

**CHEMICAL ABSTRACTS, Band 102, Nr. 15, 15.
April 1985, Seite 296, Ref. Nr. 128184e, Columbus, Ohio, US; E. BISCHOFF et al.: "Color
test for allergenic house dust. Part 2. Supplementary aspects" & Allergologie, 1985,
8(1), 36-8**

(73) Patentinhaber: **Werner & Mertz GmbH
Ingelheimstrasse 1-3
W-6500 Mainz 1(DE)**

(72) Erfinder: **Bischoff, Edelbert, Dr.
Leibnizstrasse 52
W-6719 Kirchheim-Bolanden(DE)**
Erfinder: **Schirmacher, Wolfgang, Dipl.-Chem.
Rilkeallee 28
W-6500 Mainz 31(DE)**

(74) Vertreter: **Zumstein, Fritz, Dr. et al
Dr. F. Zumstein sen Dr. E. Assman Dipl.-Ing.
F. Klingseisen Dr. F. Zumstein jun. Bräuhausstrasse 4
W-8000 München 2(DE)**

EP 0 152 068 B1

## Beschreibung

Dem Auftreten von Hausstaubmilben (Dermatophagoides pteronyssinus, farinae etc.) wird in neuerer Zeit zunehmend Aufmerksamkeit gewidmet (vgl. G Hoffmann in "Luftqualität in Innenräumen" Gustav Fischer Verlag Stuttgart - New York 1982 Seite 385 bis 401). Grund dafür ist, daß festgestellt wurde, daß ein beträchtlicher Teil der Bevölkerung - man rechnet mit etwa 5 % - unter asthmatischen Erscheinungen leidet, die auf Bestandteile des Hausstaubes zurückzuführen sind.

Bei diesen Bestandteilen handelt es sich hauptsächlich um den Kot der obengenannten Hausstaubmilben sowie um Bruchstücke abgestorbener Milben. Im Darmtrakt der lebenden und abgestorbenen Milben sind die die Gesundheitsstörungen auslösenden Allergene enthalten (vgl. E Petri et al in Allergologie, Jg. 5, Nr. 3/1982 Seite 109 bis 119). Eine lebende Milbe selbst enthält nur etwa ein Tausendstel der Allergenmenge, die sie in der Zeit ihres Lebens erzeugt. Der Kot selbst ist vielfach von Pilzen besiedelt, die ihrerseits ebenfalls Allergene hervorbringen können. Die Hauptmenge der Allergene befindet sich in den Staubablagerungen, und zwar auch dann noch, wenn die ursprüngliche Milbenpopulation durch Bekämpfungsmaßnahmen abgetötet bzw. reduziert wurde.

Aufenthaltsorte der Hausstaubmilben sind Polstermöbel, Betten, Fußböden und Wände, insbesondere wenn sie mit textilen Materialien überzogen sind. Flächenmäßig gesehen dürften Teppichböden die größte Rolle spielen.

Von den Fußböden und Polstermöbeln wird durch Begehen und Benutzen Feinststaub aufgewirbelt, der sich oft an unzugänglichen Stellen, die weitgehend frei von Luftzug sind, z.B. hinter Schränken, ansammelt. Auch beim Staubsaugen selbst wird dieser Feinststaub zum Teil aufgewirbelt und gelangt so auch an Stellen, die selbst nicht von Milben besiedelt sind. In allen Fällen führt erneutes Aufwirbeln zu erneuten Belästigungen der Bewohner der betreffenden Räume.

Die Beseitigung der Ursachen für die Hausstaub-Allergie hat zur Voraussetzung, daß die Ursache erkannt wird. Denn es gibt auch andere Allergien, wie z.B. gegen Pollen (Heuschnupfen). Die Hausstaub-Allergie belästigt jedoch die davon betroffenen Menschen das ganze Jahr über, da Milbenkot und Milbenreste praktisch ununterbrochen vorhanden sind. Arzt und Patient stehen deshalb immer wieder vor der Frage, ob ein Milbenbefall vorliegt.

Aufgrund des derzeitigen Standes der Technik gibt es als Nachweismethode nur das mikroskopische Erkennen der Milben selbst. Dieses Verfahren ist jedoch mit so großen Schwierigkeiten verbunden, daß es nur von wenigen darauf spezialisierten Fachlaboratorien durchgeführt werden kann. Dazu sind die Entnahme der Staubprobe, der Versand derselben unter vorgeschriebenen Bedingungen und dann die Untersuchung im Laboratorium notwendig. Da die Milben neben dem sonstigen Schmutz nicht zu erkennen sind, müssen sie von der Staubprobe abgetrennt und dann identifiziert werden.

Das bewährteste bekannte Abtrennverfahren besteht in der Flotation, verbunden mit dem manuellen Isolieren der aufschwimmenden Tiere sowie deren Identifikation unter dem Mikroskop (vgl. J.E.M.H. van Bronswijk et al in Allergie und Immunol. 24 (1978) Seite 18 bis 28). Abgesehen von der Tatsache, daß dies nur erfahrene Fachleute durchführen können, ist hervorzuheben, daß ein beträchtlicher Zeitaufwand erforderlich ist, um eine Probe zu bearbeiten; hinzukommen der Versand und das Risiko der Proben-Veränderung während des Transportes.

Allen Identifikationsverfahren, die von Vorhandensein der Milben selbst ausgehen, haftet der Nachteil an, daß Aussagen über die Belastung des Staubes durch Allergene, die die eigentliche Krankheitsursache darstellen, nicht zu erhalten sind.

Staubproben, insbesondere solche feinster Struktur, wie sie sich nach den Aufwirbeln aus der Luft ablagern, können besonders reich an Allergenen sein und wenige oder gar keine Milben enthalten.

Aufgabe gemäß europäischer Anmeldung EP-A-0 144 820 (Priorität: 06.12.83, Veröffentlichungstag: 19.06.85) war es daher, ein einfaches und zuverlässiges Nachweisverfahren zum Erkennen von allergenhaltigem Hausstaub zu schaffen.

Gemäß dieser Anmeldung wurde festgestellt, daß ein (wesentlich einfacherer) Nachweis geführt werden kann, der charakteristisch für die Belastung des Hausstaubes mit Ausscheidungsprodukten der Hausstaubmilben ist, unabhängig davon, ob nun lebende bzw. tote Milben im Moment des Nachweises vorhanden sind oder nicht.

Der Nachweis knüpft an Bestandteile der Ausscheidungsprodukte der Milben an, wie sie sich sowohl im Verdauungstrakt der Milben selbst als auch in deren Exkrementen befinden.

Dieser Nachweis ist problemlos sowohl von Fachleuten als auch Laien durchführbar und gibt nicht nur eine Aussage über die Anwesenheit von Exkrementen der Hausstaubmilben im Hausstaub, sondern ermöglicht auch eine Beurteilung des momentanen bzw. früheren Befalles mit Milben.

Das Nachweisverfahren gemäß europäischer Anmeldung EP-A-0 144 820 (Priorität: 06.12.83, Veröffentlichungstag: 19.06.85) Feststellung des Vorkommens von allergenhaltigem Hausstaub, wie er sich bei oder nach dem Auftreten von Hausstaubmilben ergibt, besteht darin, daß man Proben von vorkommendem Staub, wie er in Gebäuden anfällt, in wäßrig-alkoholischer Alkalimetallhydroxid-Lösung gründlich suspendiert und mit dem entstehenden Extrakt eine Farbreaktion mit Hilfe einer aromatischen Diazoverbindung durchführt, wobei die Farbreaktion hinsichtlich ihres Auftretens als Indiz für die Belastung des Staubes mit Hausstaubmilben-Rückständen bewertet wird.

Im einzelnen ist für die Durchführung des Nachweisverfahrens gemäß europäischer Anmeldung EP-A-0 144 820 (Priorität: 06.12.83, Veröffentlichungstag: 19.06.85) folgendes bemerken. Als Alkohol für die eingesetzte wäßrig-alkoholische Lösung verwendet man bevorzugt einen mit Wasser unbegrenzt mischbaren Alkohol, wie z.B. niedere einwertige aliphatische Alkohole. Dabei ist Methanol besonders bevorzugt; aber auch Ethanol kann eingesetzt werden. Das Gewichtsverhältnis von Alkohol zu Wasser in der wäßrig-alkoholischen Lösung kann variieren und beträgt im allgemeinen (10 bis 95) : (90 bis 5). Besonders bewährt hat sich ein Gewichtsverhältnis von Alkohol zu Wasser von (50 bis 90) : (50 bis 10), speziell von (75 bis 90) : (25 bis 10).

Das in der wäßrig-alkoholischen Lösung eingesetzte Alkalimetallhydroxid ist bevorzugt Kaliumhydroxid und Natriumhydroxid. Aber auch Lithiumhydroxid kann eingesetzt werden. Die Menge des Alkalimetallhydroxids in der wäßrig-alkoho lischen Lösung kann variieren; im allgemeinen beträgt sie 0,1 bis 50 Gew.-%. Vorteilhaft ist ein Alkalimetallhydroxidgehalt von 0,1 bis 20 Gew.-%, bevorzugt 0,1 bis 10 Gew.-%. Ganz speziell bevorzugt beträgt der Alkalimetallhydroxidgehalt in der wäßrig-alkoholischen Lösung etwa 4 bis 5 Gew.-%.

Für die Farbreaktion eignen sich grundsätzlich alle Verbindungen, die zusammen mit dem Milbenrückstände enthaltenden Staub Farbreaktionen ergeben. Die im vorliegenden Fall angewandte Reaktion beruht auf der Fähigkeit der Milbenrückstände (Milbenkotreste und Bruchstücke abgestorbener Milben), mit aromatischen Diazoverbindungen eine Farbreaktion zu ergeben. Als aromatische Diazoverbindung ist daher jede aromatische Diazoverbindung geeignet, die mit der Extraktionslösung (im Falle des Vorhandenseins von Hausstaubmilbenrückständen) eine Farbreaktion eingeht. Als aromatische Diazoverbindung besonders bewährt hat sich Diazosulfanilsäure (4-Diazobenzosulfonsäure), die eine ziegelrote Färbung ergibt.

Die vorliegende Anmeldung betrifft nun eine besondere Weiterbildung des Verfahrens gemäß der genannten älteren Anmeldung EP-A-0 144 820 (Priorität: 06.12.83, Veröffentlichungstag: 19.06.85). Diese Weiterbildung besteht darin, daß man zur Durchführung der Farbreaktion zwischen dem Extrakt und der aromatischen Diazoverbindung ein mit der Diazoverbindung und gegebenenfalls mit Stärke präpariertes Papier verwendet.

Bei dem vorliegenden erfindungsgemäßen Nachweisverfahren geht man zweckmäßig in der Weise vor, daß man eine gewonnene Staubprobe (nach Entfernung der Grobanteile) mit der wäßrig-alkoholischen Alkalimetallhydroxid-Lösung durchtränkt. Dabei kann das Volumenverhältnis der Staubprobe zur Extraktionslösung variieren. Im allgemeinen beträgt dieses Volumenverhältnis der Staubprobe zur Extraktionslösung (0,1 bis 2) : (1). Besonders bewährt hat sich ein Volumenverhältnis von 0,5 : 1.

Die Staubprobe wird mit der Extraktionslösung versetzt und durch Schütteln oder Rühren gut vermischt. Die erhaltene Extraktsuspension läßt man stehen, wobei sich der Staub im allgemeinen von der Extraktionslösung absetzt (wenige Minuten).

Die Extraktionslösung wird sodann mit dem mit der aromatischen Diazoverbindung (und gegebenenfalls mit Stärke) präparierten Papier in Kontakt gebracht. Vorteilhaft erfolgt dies durch Eintauchen des präparierten Papiers in die Extraktionslösung.

Aufgrund der auf dem präparierten Papier vorhandenen aromatischen Diazoverbindung, z.B. Diazosulfanilsäure, entsteht bei Anwesenheit von Milbenexkrementen eine ziegelrote Färbung des Papiers, die, wie die Anmelderin festgestellt hat, in ihrer Intensität proportional zu der in der Staubprobe vorhandenen Milbenkotkonzentration ist.

Die bevorzugte erfindungsgemäße Ausführungsform besteht darin, einen Papierstreifen, z.B. Filterpapier, mit der aromatischen Diazoverbindung (z.B. mit ca. 5 Gew.-% Diazoverbindung, bezogen auf das Papier) zu präparieren und den so präparierten Papierstreifen direkt in die überstehende Extraktionslösung zu tauchen, wobei die Farbreaktion dann auf dem Papier erfolgt. Die Benutzung ist vergleichbar mit derjenigen des pH-Indikatorpapiers. Unter definierten Nachweisbedingungen kann man die Farbentwicklung mit einer vorgegebenen Farbskala vergleichen. Mittels eines solchen Vergleichs gegenüber einer standardisierten Farbskala lassen sich dann Aussagen über die Intensität an Hausstaubmilbenrückständen im Hausstaub machen.

Im Falle der erwähnten Arbeitsweise, d.h. der Präparierung eines Papierstreifens mit der aromatischen Diazoverbindung, hat es sich als besonders vorteilhaft herausgestellt, wenn die aromatische Diazoverbindung zusammen mit Stärke auf den Papierstreifen aufgebracht wird. Dadurch läßt sich bei der Farbreaktion eine noch gleichmäßigere Färbung auf dem derart präparierten Papierstreifen erzielen. Die Menge der zusammen mit der aromatischen Diazoverbindung verwendeten Stärke kann variieren; jedoch beträgt sie im allgemeinen etwa 50 Gew.-% der Menge der aromatischen Diazoverbindung. In diesem Zusammenhang hat die Anmelderin in überraschender Weise festgestellt, daß ein mit der aromatischen Diazoverbindung präparierter Papierstreifen (z.B. ein mit etwa 50 mg Diazosulfanilsäure, pro g Papier, und mit etwa 25 mg Stärke, pro g Papier, präparierter Papierstreifen) ohne jedes Risiko über längere Zeit hinweg (mehrere Monate) gefahrlos und stabil gelagertwerden kann; dies ist im Hinblick auf die bekannten Eigenschaften aromatischer Diazoverbindungen durchaus unerwartet.

Dem Nachweisverfahren selbst liegen einige spezielle und zum Teil überraschende Befunde zugrunde:

1) Um die für den Farbnachweis relevanten Kotbestandteile bei Normaltemperatur mit der wäßrig-alkoholischen Extraktionslösung schnell und erschöpfend zu extrahieren, ist eine Alkalimetallhydroxid -Lösung (z.B. von KOH oder von NaOH) in der angegebenen Konzentration nötig (eine entsprechend konzentrierte Soda lösung, wie sie eigentlich gemäß der nachfolgenden Ziffer 2) nötig wäre, führt nicht zum Erfolg).

2) Die Farbreaktion mit der aromatischen Diazoverbindung, insbesondere mit Diazosulfanilsäure, würde an sich am besten in nur wäßriger, sodaalkalischer Lösung verlaufen. (In nur wäßriger Alkalimetallhydroxid-Lösung, z.B. von KOH oder NaOH, findet die gewünschte Farbreaktion - wie die Anmelderin festgestellt hat - nicht statt.) Ausgehend von dieser besonderen Problematik im Hinblick auf die Gestaltung eines einfachen Nachweisverfahrens, wurde nun aber überrraschend gefunden, daß bei Einsatz einer Extraktionslösung von Alkalimetallhydroxid in einem Lösungsmittelgemisch von Alkohol und Wasser, insbesondere in einem Gemisch, bei dem das Gewichtsverhältnis von Alkohol zu Wasser (50 bis 90) : (50 bis 10) beträgt, einerseits die für den Farbnachweis relevanten Kotbestandteile praktisch quantitativ extrahiert werden, und daß andererseits in derselben Extraktionslösung bei Kontakt mit dem präparierten Papier (mit der aromatischen Diazoverbindung, insbesondere Diazosulfanilsäure) die gewünschte Farbreaktion stattfindet. Außerdem verbessert der Alkohol die Benetzung der Staubproben und bewirkt gleichzeitig eine schnelle und gleichmäßige Farbverteilung auf dem Nachweispapier.

Nachstehend werden weitere Einzelheiten zur Durchführung des Nachweisverfahrens angegeben. Das gefundene Analysenverfahren auf Befall von Hausstaub mit allergenhaltigen Rückständen von Hausstaubmilben läßt sich mannigfaltig ausgestalten. Dies gilt sowohl für die Probenentnahme des Staubes, als auch für das Zusammenbringen des Staubes mit der Extraktionslösung, als auch für den Ablauf der Farbreaktion.

Im Sinne einer besonders effizienten Staubgewinnung kann in die Ansaugröhre eines üblichen Staubsaugers ein Filter (insb. Stoff- oder Papier-Filter) eingelegt werden, z.B. in Form eines kleinen Tuches (insbesondere Taschentuches). Es hat den Vorteil, daß der Feinstaub gezielt und konzentriert gesammelt wird. Bei der Probenentnahme selbst ist zu berücksichtigen, daß Bodenflächen, die des öfteren abgesaugt werden, eine für den Nachweis ausreichende Staubmenge eventuell nicht mehr enthalten können; in diesem Falle führt man dann zweckmäßig das Absaugen an weniger zugänglichen Stellen durch. Besonders vorteilhaft erfolgt die Staubgewinnung durch Absaugen von Polstermöbeln und Betten.

Für die Extraktion der Staubproben eignen sich besonders kleine verschließbare Glas- und Kunststoffbehälter mit einem Fassungsvermögen von einigen Millilitern (z.B. 1 bis 10 Milliliter), die mit Markierungen zur Volumendosierung der Staubprobe und der Extraktionslösung versehen sind. In speziellen Fällen kann die Extraktion der Staubprobe aber auch z.B. auf einem Uhrglas erfolgen.

Eine zusätzliche Vereinfachung kann man - bei der vorliegenden Weiterbildung des Verfahrens der europäischen Anmeldung EP-A-0 144 820 (Priorität: 06.12.83, Veröffentlichungstag: 19.06.85) - dadurch erzielen, daß man die (dünnflüssige und z.T. leicht flüchtige) Extraktionslösung verdickt, beispielsweise mit einem üblichen Verdickungsmittel, insbesondere Celluloseether. Bevorzugt wird die so verdickte Extraktionslösung in kleinen Kunststoffbehältern vorgelegt. Nach dem Einrühren der dosierten Staubprobe erhält man eine breiige Masse. Für den Farbnachweis drückt man das präparierte Papier (z.B. das mit der aromatischen Diazoverbindung präparierte Filterpapier) einseitig auf die breiige Probe in dem Behälter, wobei nach dem Abheben des Papiers ein Teil der feuchten Staubmasse daran haften bleibt. Die Beurteilung der Färbung erfolgt über die andere (unverschmutzte) Seite des Papiers. Bei dieser Ausführungsform der erfindungsgemäßen Weiterbildung wird besonders die Verdunstung und die Gefahr des Verspritzens verringert.

Die Effizienz des erfindungsgemäßen Verfahrens wird durch den folgenden Vergleich illustriert: An 20 Staubproben, die von Ärzten aus verschiedenen Häusern mit Verdacht auf Hausstaub-Allergie eingesandt wurden, erfolgte zunächst eine biologische Beurteilung unter Anwendung des Flotationsverfahrens (gemäß Stand der Technik, wie oben beschrieben). Diese Bewertung der Proben ergab einen mittelstarken, schwachen bzw. keinen Befall.

Danach wurden mit den gleichen Proben erfindungsgemäße Bewertungen durchgeführt, und zwar von einer Person, die keine Kenntnis von der (vorherigen) biologischen Beurteilung hatte. Die erfindungsgemäßen Farbreaktionen zeigten mittleren, schwachen und keinen Befall. Beim Vergleich dieser Ergebnisse mit dem Laborjournal, in dem die (vorherigen) biologischen Befunde registriert waren, ergab sich völlige Übereinstimmung.

**Beispiel 1**

Man versieht einen normalen Staubsauger mit einem neuen Staubbeutel und saugt den zu untersuchenden Textilbereich (z.B. Teppiche, Polstermöbel, Deckbetten, Matratzen) gründlich ab. Nach Beendigung des Absaugens entleert man den gesamten Staubbeutelinhalt auf ein Blatt Papier und entfernt durch leichtes Abklopfen und Abheben die Grobanteile. Der auf dem Papier verbleibende Feinstaub wird für den Farbnachweis verwendet.

Für den Farbnachweis dosiert man die Feinstaubprobe (ca. 2 ml) in einen Glas- oder Kunststoffzylinder von ca. 8 ml Inhalt (Durchmesser ca. 1,5 cm). Dazu dosiert man ca. 4 ml einer wäßrigmethanolischen Extraktionslösung, bei der das Gewichtsverhältnis von Methanol zu Wasser 75 : 25 beträgt und in der 5 Gew.-% KOH gelöst sind, und schüttelt das verschlossene Gefäß ca. 10 Sekunden. Nach etwa 3 Minuten taucht man einen Teststreifen aus Filterpapier, der mit 50 mg Diazosulfanilsäure und 25 mg wasserlöslicher Stärke (jeweils pro g Papier) präpariert war, kurz in die vom Staub abgesetzte Extraktionslösung (entsprechend der Anwendung eines pH-Papiers). Nach spätestens ca. 15 Sekunden zeigt sich bei Milbenbefall eine ziegelrote Färbung (diese bleibt bei Abwesenheit von Milbenrückständen aus).

**Beispiel 2**

Die Staubprobenahme erfolgt analog dem Beispiel 1.

Man dosiert die Feinstaubprobe auf ein Uhrglas von ca. 6 cm Durchmesser, fügt die geeignete Menge Extraktionslösung (Methanol/Wasser-Gemisch im Gewichtsverhältnis 75 : 25, worin 4 Gew.-% NaOH gelöst sind) hinzu und verrührt den Staub kurzzeitig mit der Extraktionslösung. Daran anschließend drückt man einen gemäß Beispiel 1 mit Diazosulfanilsäure präparierten Papierstreifen einseitig gegen die breiige Masse. Nach spätestens ca. 15 Sekunden zeigt sich bei Milbenbefall eine ziegelrote Färbung auf der unverschmutzten Papierseite.

**Ansprüche**

1. Verfahren zur Feststellung des Vorkommens von allergenhaltigem Hausstaub, wie er sich bei oder nach Auftreten von Hausstaubmilben ergibt,
bei dem man Proben von in Gebäuden vorkommendem Staub in wäßrig-alkoholischer Alkalimetallhydroxid-Lösung gründlich suspendiert und mit dem entstehenden Extrakt eine Farbreaktion mit Hilfe einer aromatischen Diazoverbindung durchführt, wobei die Farbreaktion hinsichtlich ihres Auftretens als Indiz für die Belastung des Staubes mit Hausstaubmilben-Rückständen bewertet wird, und wobei man zur Durchführung der Farbreaktion zwischen dem Extrakt und der aromatischen Diazoverbindung ein mit der Diazoverbindung und gegebenenfalls mit Stärke präpariertes Papier verwendet.

2. Verfahren nach Anspruch 1, dadurch gekennzeichnet, daß eine Extraktionslösung eingesetzt wird, die mit einem üblichen Verdickungsmittel verdickt worden ist.

3. Verfahren nach einem der vorhergehenden Ansprüche, dadurch gekennzeichnet, daß eine aufgetretene Farbreaktion hinsichtlich der Intensität der Färbung mit einer standardisierten Farbskala verglichen und aufgrund dieses Vergleichs die Konzentration an Milbenrückständen beurteilt wird.

4. Verfahren nach einem der vorhergehenden Ansprüche, dadurch gekennzeichnet, daß der Alkalimetallhydroxid-Gehalt in der wäßrig-alkoholischen Lösung 0,1 bis 50 Gew.-% beträgt.

5. Verfahren nach einem der vorhergehenden Ansprüche, dadurch gekennzeichnet, daß das Gewichtsverhältnis von Alkohol zu Wasser in der Extraktionslösung 10 bis 95 : 90 bis 5 beträgt.

6. Verfahren nach einem der vorhergehenden Ansprüche, dadurch gekennzeichnet, daß das Volumenverhältnis der Staubprobe zur Extraktionslösung 0,1 bis 2 : 1 beträgt.

**Claims**

1. Method of detecting the presence of allergen-containing house dust such as occurs during or after the appearance of house dust mites, wherein samples of dust found in buildings are suspended thoroughly in a aqueous-alcoholic alkali metal hydroxide solution and a dye reaction is carried out with the resulting extract using an aromatic diazo compound, the occurrence of dye reaction being evaluated as an indication of the presence of house dust mite residues in the dust, the dye reaction between the extract and the aromatic diazo compound being carried out using a piece of paper pretreated with the diazo compound and optionally with starch.

2. Method according to claim 1, characterised in that an extraction solution is used which has been thickened with a conventional thickener.

3. Method according to one of the preceding claims, characterised in that, once a dye reaction has occurred, the intensity of the coloration is compared with a standardised colour scale and the concentration of mite residues is assessed on the basis of this comparison.

4. Method according to one of the preceding claims, characterised in that the content of alkali metal hydroxide in the aqueous-alcoholic solution is 0.1 to 50 by weight.

5. Method according to one of the preceding claims characterised in that the weight ratio of alcohol to water in the extraction solution is 10 to 95: 90 to 5.

6. Method according to one of the preceding claims characterised in that the ratio by volume of the dust sample to the extraction solution is 0.1 to 2: 1.

**Revendications**

1. Procédé pour la constatation de la présence de poussière domestique contenant des allergènes, telle qu'elle se présente lors de l'apparition d'acariens de poussière domestique ou après, dans lequel on met totalement en suspension en solution hydroalcoolique d'hydroxyde de métal alcalin des échantillons de poussière présente dans des bâtiments et on effectue, avec l'extrait résultant, une réaction colorée à l'aide d'un composé diazoïque aromatique, la réaction colorée étant appréciée, en ce qui concerne son apparition, en tant qu'indice de la charge de la poussière en résidus d'acariens de poussière domestique et un papier préparé avec le composé diazoïque et éventuellement avec de l'amidon étant utilisé pour effectuer la réaction colorée entre l'extrait et le composé diazoïque aromatique.

2. Procédé selon la revendication l, caractérisé en ce que l'on met en oeuvre une solution d'extraction qui a été épaissie avec un agent épaississant usuel.

3. Procédé selon l'une des revendications précédentes, caractérisé en ce qu'une réaction colorée qui a lieu est comparée, en ce qui concerne l'intensité de la coloration, avec une échelle colorimétrique standardisée et que la concentration en résidus d'acariens est évaluée sur la base de cette comparaison.

4. Procédé selon l'une des revendications précédentes, caractérisé en ce que la teneur en hydroxyde de métal alcalin de la solution hydroalcoolique est de 0,l à 50% en poids.

5. Procédé selon l'une des revendications précédentes, caractérisé en ce que le rapport pondéral d'alcool à eau dans la solution d'extraction est de l0 à 95 : 90 à 5.

6. Procédé selon l'une des revendications précédentes, caractérisé en ce que le rapport en volume de l'échantillon de poussière à la solution d'extraction est de 0,l à 2 : l.